# EUROPEAN PATENT SPECIFICATION

(11) **EP 2 905 000 B1**
(45) Date of publication and mention of the grant of the patent: **28.12.2016**
(21) Application number: 15151570.7
(22) Date of filing: 19.01.2015
(51) Int. Cl.: A61F 13/15

(54) **Method and apparatus for making an absorbent structure comprising channels**
Verfahren und Vorrichtung zum Herstellen von absorbierenden Strukturen mit Kanälen
Procédé et appareil de fabrication d'une structure absorbante comprenant des canaux

(30) Priority: 11.02.2014 EP 14154593
(43) Date of publication of application: 12.08.2015
(73) Proprietor: The Procter & Gamble Company, Cincinnati, OH 45202 (US)
(72) Inventor: Jackels, Hans Adolf, 53881 Euskirchen (DE)
(74) Representative: Mather, Peter Geoffrey

(56) References cited:
- EP-A1- 2 532 329
- EP-A1- 2 679 209
- JP-A- 2002 065 718
- US-A1- 2010 051 166

## Description

### FIELD OF THE INVENTION

The present invention relates to an apparatus and method for making an absorbent structure with channels embedded within the absorbent structure. The channels are strips that are free of absorbent material.

### BACKGROUND OF THE INVENTION

Absorbent articles, such as diapers and sanitary napkins, absorb and contain body exudates. They also are intended to prevent body exudates from soiling, wetting, or otherwise contaminating clothing or other articles, such as bedding, that come in contact with the wearer. A disposable absorbent article, such as a disposable diaper, may be worn for several hours in a dry state or in a urine-loaded state. Accordingly, efforts have been made toward improving the fit and comfort of the absorbent article to the wearer, both when the article is dry and when the article is fully or partially loaded with liquid exudate, while maintaining or enhancing the absorbing and containing functions of the article.

Efforts have also been made to make absorbent article thinner when dry, to improve the comfort of such articles.

Some absorbent articles, like diapers, contain absorbent material such as super absorbent polymers that absorbs very high quantities of liquid and causes the absorbent article to swell significantly. Such articles will thus increase significantly in volume during use, and sometimes in particular in the crotch area between the wearer's legs, which may render the article uncomfortable.

There is thus still a need to further improve the fit of such articles and/or the liquid transportation away from the crotch. There is also a need to reduce the usage of absorbent material in such articles.

There is also still a need to further reduce the chance of leakage and to improve the efficiency of absorbency of an absorbent article, such as a diaper.

It has also been found that improved liquid transportation can be achieved by the provision of transportation channels for distributing liquid in the absorbent article, e.g. the absorbent structure thereof. Furthermore, it has surprisingly been found that the amount of absorbent material can be reduced hereby, whilst maintaining the performance. It has been found that improved fit can be obtained by providing absorbent articles with absorbent structures whereby the absorbent material is structured in machine direction, optionally with areas that comprise less or no absorbent material, for improved bending flexibility in use (in the direction corresponding to the machine direction).

EP-A-2 532 329, published on December 12th 2012, discloses an apparatus and method for producing an absorbent structure comprising at least two supporting sheets and a layer of absorbent material deposited, preferably by a printing process, onto at least one of the supporting sheets. It discloses a separate pressure roll which may selectively apply pressure only onto the channel area of the absorbent structure. The pressure means may comprise raised mating strips. Similarly US 2010/051166, published on March 4th 2010, and EP 2 679 209, published on January 1st 2014, also disclose an apparatus and method for producing an absorbent structure comprising at least two supporting sheets and a layer of absorbent material deposited, preferably by a printing process, onto at least one of the supporting sheets.

JP 2002 065718, published on March 5th 2002, provides a cold insulant which is formed by sealing a high-polymer water absorptive resin into a gap formed by laminating a permeable base fabric and a thermoplastic resin film having plural projecting parts.

The present invention relates to an apparatus for making an absorbent structure for an absorbent article, comprising a supporting sheet and thereon an absorbent layer, the absorbent layer comprising an absorbent material, the apparatus comprising:
a) transfer means for transferring first and second supporting sheets to first and second moving endless surfaces;
b) a feeder for feeding the absorbent material onto at least the first supporting sheet at a depositing point on the first moving endless surface, the absorbent material forming absorbent regions upon the supporting sheet, and one or more channels between the absorbent regions, the channels being free of absorbent material;
c) an adhesive applicator for applying adhesive to at least one of the first and second supporting sheets, at least within the region of the channels.

The present invention further relates to a method for making an absorbent structure comprising a supporting sheet and thereon an absorbent layer of absorbent material, the method comprising the steps of:
a) transferring first and second supporting sheets to first and second moving endless surfaces;
b) feeding the absorbent material onto at least the first supporting sheet at a depositing point on the first moving endless surface, the absorbent material forming absorbent regions and one or more channels between the absorbent regions, the channels being free of absorbent material;
c) applying adhesive to at least one of the first and second supporting sheets, at least within a region of the channels.

The present invention addresses the problem of ensuring accurate registration between the pattern of regions free of absorbent material formed in the depositing or printing step, and the pressure applied by separate pressure rolls in a downstream gluing step.

The present invention combines the depositing or printing step and the pressure step which forms the channels by adhesion onto a single moving endless surface, e.g. a roll.

### SUMMARY OF THE INVENTION

According to the present invention a first moving endless surface is provided which has one or more longitudinally extending first mating strips, and the second moving endless surface is provided which has corresponding longitudinally extending second mating strips. Pressure is applied to the first and second supporting sheets, between the first and second mating strips, at least within a part of the area of the channels, so as to adhere together the first and second supporting sheets and form channels that are free of absorbent material.

### BRIEF DESCRIPTION OF THE DRAWINGS

Fig. 1A is a top view of a reservoir 25.
Fig. 1B is a top view of an alternative reservoir 25.
Fig. 2 is a side view of an apparatus of the present invention, or used in the method of the invention.
Fig. 3 is a partial perspective view of an apparatus of the present invention, or used in a method of the invention.
Fig. 4 is a partial perspective view of an alternative apparatus of the present invention, or used in an alternative method of the invention.

### DETAILED DESCRIPTION OF THE INVENTION

As summarized above, this invention encompasses a method and apparatus for making an absorbent structure useful for an absorbent article comprising absorbent material, preferably comprising at least, or only, particulate superabsorbent polymer material. Embodiments of such method and apparatus and resulting absorbent structures and absorbent articles are further described herein, after the following definitions.

### Definitions

"Absorbent structure" refers to a three-dimension structure with a longitudinal dimension and, perpendicular thereto, a transverse dimension, and, perpendicular to both, a height dimension, and that comprises at least an absorbent material and a supporting sheet, and that is useful in an absorbent article.

"Absorbent layer" refers to a three dimensional layer of absorbent material, formed by deposition of absorbent material onto the supporting sheet.

"Absorbent material" refers to a material or mixture of materials that can absorb and retain bodily fluids; it typically includes or consists of "superabsorbent polymer material". "Superabsorbent polymer material" (also known as "absorbent gelling material," or "AGM," or "superabsorbent,") refer to polymeric material that can absorb at least 10 times (and typically at least 15 times, or at least 20 times) their weight of an aqueous 0.9% saline solution as measured using the Centrifuge Retention Capacity test (Edana 441.2-02)., i.e. having a CRC of at least 10 g/g, and typically at least 15 g/g or at least 20 g/g.

"Absorbent article" refers to a device that absorbs and contains body exudates, and, more specifically, refers to devices that are placed against or in proximity to the body of the wearer to absorb and contain the various exudates discharged from the body. Absorbent articles may include adult and infant diapers, including pants, such as infant training pants and adult incontinence undergarments, and feminine hygiene products, such as sanitary napkins and panty-liners and adult incontinent pads, and breast pads, care mats, bibs, wound dressing products, and the like. Absorbent articles may further include floor cleaning articles, food industry articles, and the like. As used herein, the term "body fluids" or "body exudates" includes, but is not limited to, urine, blood, vaginal discharges, breast milk, sweat and fecal matter.

"Diaper" refers to an absorbent article generally worn by infants and incontinent persons about the lower torso so as to encircle the waist and legs of the wearer and that is specifically adapted to receive and contain urinary and fecal waste.

"Pant" or "training pant", as used herein, refer to diaper having a waist opening and leg openings designed for infant or adult wearers. A pant may be placed in position on the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the terms "pant" or "pants" are used herein, pants are also commonly referred to as "closed diapers," "prefastened diapers," "pull-on diapers," "training pants," and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Publication No. 2003/0233082 A1, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

A "nonwoven" is a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. The basis weight of nonwoven fabrics is usually expressed in grams per square meter (gsm).

"Particulate" is used herein to refer to a material which is in particulate form so as to be flowable in the dry state.

"Substantially cellulose free" is used herein to describe an article, such as an absorbent layer structure or core, that contains less than 5% by weight cellulosic fibers.

"Thickness" and "height" are used herein interchangeably.

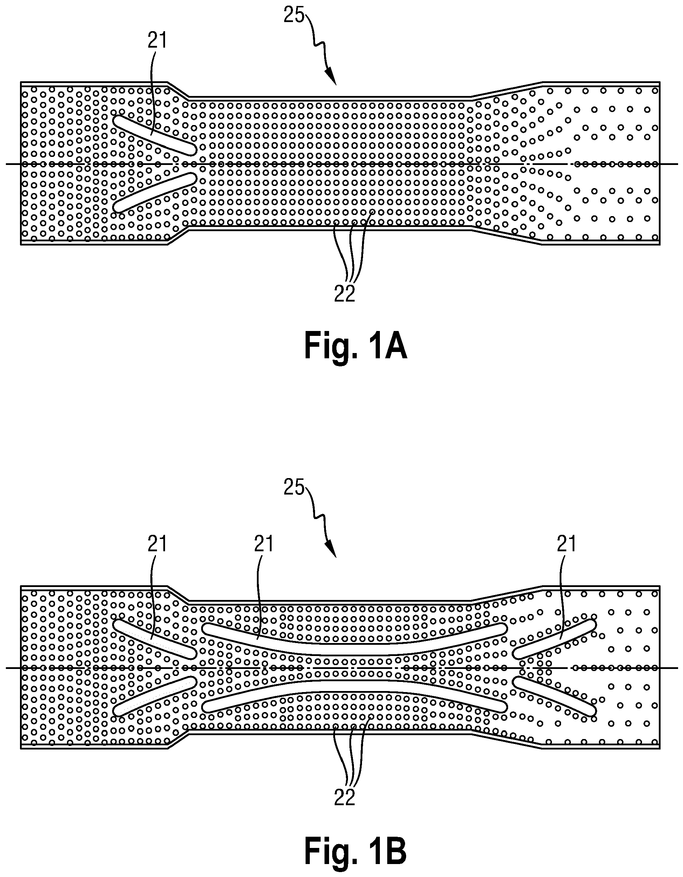

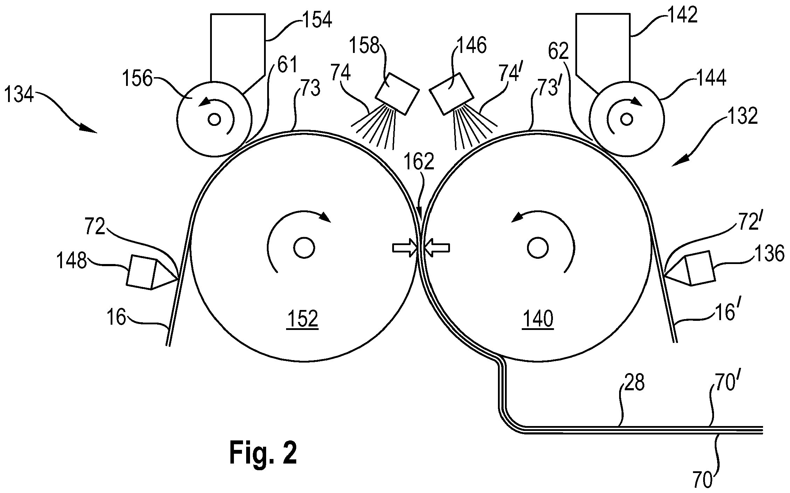

According to the present invention absorbent material 100 may be fed onto the supporting sheet 16, 16' by any suitable means including gravimetric feeding or by using a print roll. In the embodiment illustrated in Figures 1A, 1B and 2 the print roll method is used which is described in more detail below.

Figures 1A and 1B illustrate examples of patterns which provide a reservoir 25 for holding absorbent material prior to depositing, or printing the absorbent material onto a supporting web. Absorbent material is fed to the reservoir, the reservoir holds absorbent material in each of the holes or cavities 22. The reservoirs contain one or more channels 21 between the absorbent regions, the channels 21 being free of absorbent material.

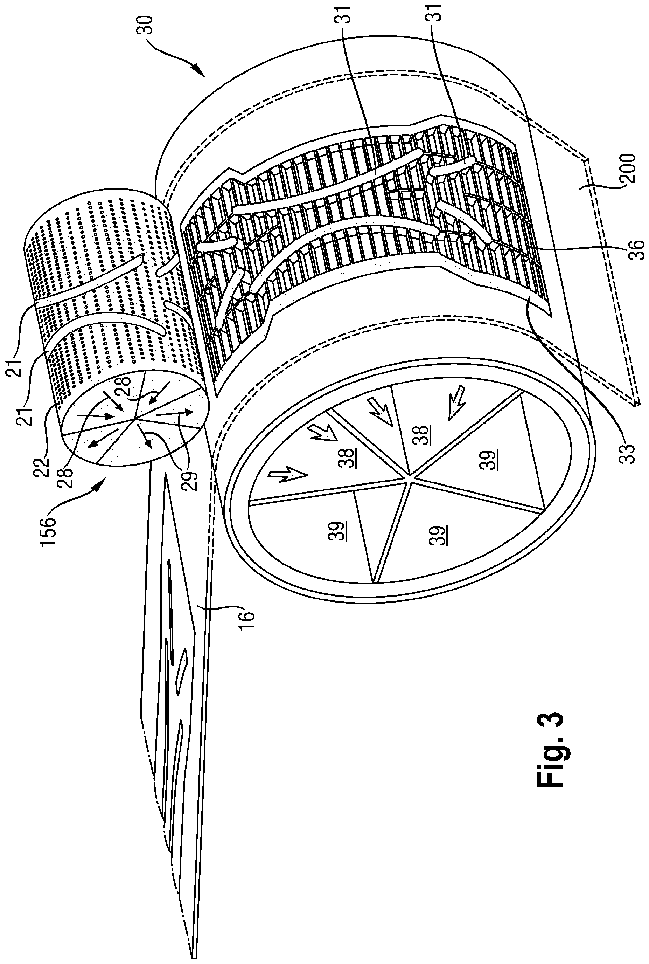

In a preferred embodiment of the invention illustrated in Figures 2 and 3 the reservoirs are disposed around the outer surface of print rolls 144, 156. The absorbent material 100 is delivered to the supporting sheet 16 by a roll 156 placed adjacent and in close proximity to the first moving endless surface 30, for example substantially above the surface. The absorbent material 100 may be deposited substantially continuously. The point or area where the absorbent material 100 leaves the roll 156 and transfers to the first moving endless surface 30 is herein referred to as the depositing point or area; and in this point or area a raised strip 21, e.g. each raised strip, mates with a mating strip 31, e.g. without direct contact.

A first printing unit 134 for making an absorbent structure n accordance with the first aspect of this invention is illustrated on the left half in Fig. 2. A second printing unit 132 for forming a second absorbent structure is represented on the right side of Fig. 2, both absorbent structures being then combined into an absorbent core in the nip 162.

The first printing unit 134 comprises an auxiliary adhesive applicator 148 for applying an auxiliary adhesive to the substrate 16, a first rotatable support roll 152 for receiving the first substrate 16, a first hopper 154 for holding the absorbent particulate polymer material, a first printing roll 156 for transferring the absorbent particulate polymer material from the hopper 154 to the substrate 16, and a thermoplastic adhesive material applicator 158 for applying the fibrous thermoplastic adhesive material 74 to the first substrate 16 and the absorbent particulate polymer material land areas thereon.

The auxiliary adhesive applicator 148 may be a nozzle system which can provide a relatively thin but wide curtain of thermoplastic adhesive material as suggested in WO2008/155699, but may instead advantageously comprise a slot coater for applying several slots of auxiliary adhesive simultaneously along the width of the substrate and fitted with a manifold to intermittently stop the delivery of the auxiliary adhesive so that there the auxiliary layer is not applied in the area of the substrate corresponding to the zones of lower absorbent material amount. The printing roll 156 and adhesive applicator 158 may be as further detailed in WO2008/155699. The absorbent structure 70 obtained by the printing unit 134 may be directly put in face to face relation with a second substrate 16', or may be combined with a second absorbent structure 70' to form an absorbent core. This second absorbent structure 70' may be formed on the second printing unit 132 as shown in Fig. 2, which may be generally identical to the first printing unit 134. The second printing unit 132 may comprise a second auxiliary adhesive applicator 136 which may be a slot coater for applying an auxiliary adhesive to the substrate 16', a second rotatable support roll 140 for receiving the substrate 16', a second hopper 142 for holding absorbent particulate polymer material, a second printing roll 144 for transferring the absorbent particulate polymer material to the substrate 16', and a thermoplastic adhesive material applicator 146 for applying the thermoplastic adhesive material 74' to the substrate 16' and the absorbent particulate polymer land areas thereon.

The radius of the roll 156 may depend on what absorbent structure is produced, e.g. what size, and for example how many structures are produced per cycle of the print roll or drum. For example, the drum/print roll may have a radius of at least 40 mm, or of at least 50 mm; it may be for example up to 300 mm, or up to 200 mm. In some embodiments, the radius of the drum/print roll is less than 50% of the radius of the first moving endless surface. In a preferred embodiment the radius of the print roll is from 60mm to 80mm.

The roll 156 may have any suitable width, but for example a width (for example in CD, hence perpendicular to MD) corresponding (substantially) to the width of the absorbent structure to be produced; this for example be at least 40 mm, or at least 60 mm, or for example up to 400 mm, or up to 200 mm.

The roll 156 may have one or more reservoirs 25 with a certain volume for receiving the absorbent material 100 therein, and transporting it to and then depositing it on the supporting sheet 16 on the first moving endless surface.

Each reservoir 25 corresponds typically to an absorbent structure to be produced, as suitable for an absorbent article.

According to the present first and second supporting sheets are carried on first and second moving endless surfaces. In Figures 2 to 4 these first and second moving endless surfaces are illustrated by drums, referred to herein as lay-down drums 140, 152. Alternatively the moving endless surfaces could be belts.

The supporting sheets 16, 16' are preferably sheets of nonwoven material. Absorbent material is deposited onto at least one, and preferably both, of the supporting sheets from the roll 156, except in the regions corresponding to the raised strips 21 and the mating strips 31.

The first roll 152 has one or more substantially longitudinally extending first mating strips 31, and the second roll 140 has corresponding longitudinally extending second mating strips. Pressure, P, is applied through the first and second mating strips to the first and second supporting sheets 16, 16' at least within a part of the area of the channels, so as to adhere together the first and second supporting sheets.

By applying pressure to the supporting sheets in this manner, between the mating strips on the first and second rolls, rather than by separate pressure rolls positioned downstream of the rolls, the problem of registration of the channel is avoided. Quality control of the absorbent structure is more easily provided, especially in high speed manufacturing processes.

In some embodiments, the first and second moving endless surfaces 140, 152 may for example have a speed of at least 1000, or at least 1200 parts per minute and/or a linear speed of at least 4.5 m/s, or at least 6 m/s, or at least 8 m/s, or at least 10 m/s.

### Lay-down Drum

Figure 3 illustrates the side-by side arrangement of the print roll 156 and the lay-down drum 152. In the embodiment illustrated in Figure 3 the drum is a rotating cylindrical drum. The outer shell rotates around a stationary inner chamber, e.g. a stator. The rotating outer shell carries one or more receptacles 33 which receive absorbent material from the print roll 156.

Preferably the receptacle 33 is at least partially air-permeable. It may have an area that serves to receive the absorbent material, and this area is substantially in air-communication with a vacuum system via vacuum chambers 38. This arrangement holds the supporting sheet 16 onto the surface of the lay-down drum 30, and may also hold the absorbent material received from the print roll 156 in place against the surface of the supporting sheet 16, if the supporting web is air permeable.

In one embodiment, not illustrated, the receptacle area 33 other than the mating strips 31 is a mesh material that has apertures, is air permeable, and is in air communication with the chambers 38 of the vacuum system.

In the embodiment of Figure 3 the surface area of the receptacle 33, other than the mating strips 31, comprises thin supports substantially in transverse direction, for supporting the supporting sheet 16. Typically the maximum dimension in the longitudinal direction is less than the average width dimension of the adjacent mating strip; for example at most 4 mm, preferably at most 3mm.

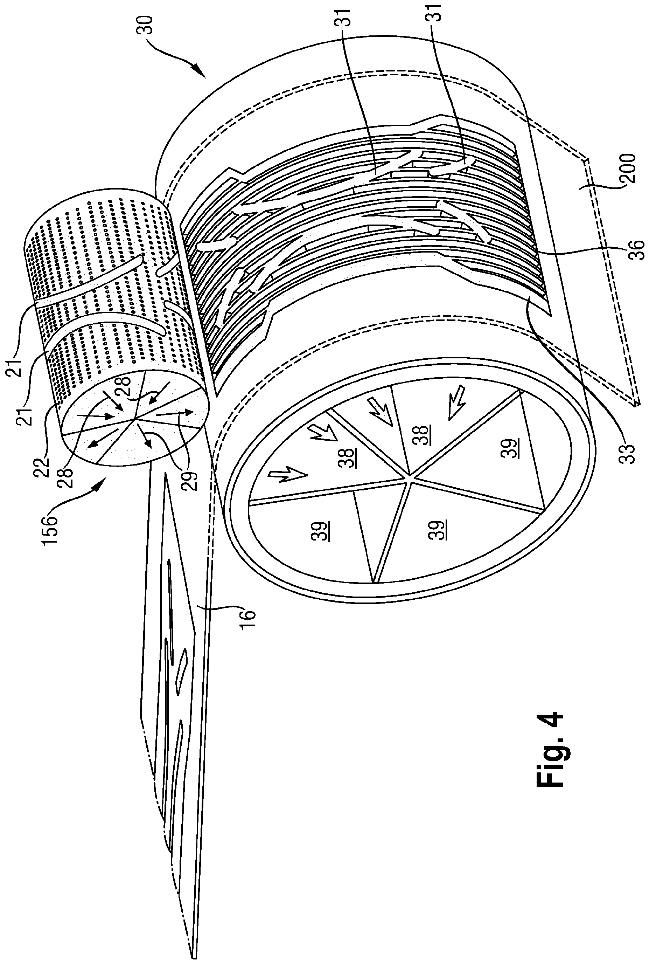

In the embodiment of Figure 4, the receptacle 33 comprises a multitude of longitudinally extending rods 36, spaced apart from one another, and typically from a neighbouring mating strip, in transverse direction. Such rods 36 may then partially form the most outer surface of the receptacle 33, so that the supporting sheet 16 is received and carried by the rods 36 and the mating strips 31.

The first mating strips 31 on the first lay-down drum correspond with second mating strips on an immediately adjacent second lay-down drum. Pressure is applied between the corresponding first and second mating strips which press against the first and second supporting webs specifically in the regions in which the absorbent structure is free of any absorbent material. The combination of pressure and adhesive material between the supporting webs causes the supporting webs to adhere together in these regions and thus forms one or more channels. The application of pressure can be accurately registered with the regions which are free of absorbent material because both the deposit of absorbent material and the application of pressure take place on the same surface, namely the receptacle 33 which is placed on the outer surface of the drum. It is preferred to avoid applying any pressure to the absorbent material which is sandwiched between the supporting webs. Such pressure could cause absorbent material particles to damage or even puncture or tear the supporting web. Such pressure acting on absorbent material particles between the mating surfaces could also cause undue wear to the mating surfaces. For this reason the area over which pressure is applies between the mating surfaces is preferably slightly narrower and preferably also slightly shorter than the region in which the absorbent material is free of any absorbent material.

Preferably the first mating strips 31 and the second mating strips are made from an elastic material, preferably silicone, . In some embodiments either the first and/or second mating strips may made from a metallic material, preferably steel.

### Process Control

In a preferred embodiment the process is controlled in order to maintain the desired pressure between the first and second mating strips. The applied pressure is an important process parameter for achieving required channel bond strength in the finished article. In the description that follows reference is made to pressure applied between two drums, however the principles may equally be applied to other forms of moving endless surfaces, such as combinations of belts and rollers.

Preferably pressure is applied by means of the first and second mating strips which form part of the outer surface of the drums. Each drum is mounted on a shaft, and each shaft is rotated at a set speed by means of a drive, for example an electric motor. Each shaft is supported in a backplate which, in turn, is mounted directly or indirectly in the machine frame. Preferably the backplates can be moved laterally with respect to each other so that the shafts are moved either closer together or further apart. Most preferably one of the backplates is moveable whilst the other backplate is fixed relative to the machine frame. This enables the pressure between the first and second mating strips to be increased or decreased as required by moving the shafts closer together or further apart.

The preferred process pressure between the first and second mating strips 31, 32 is from 0.3 to 0.8 MPa.

The drums are mounted onto the shaft and the free end of the shaft is referred to as the outside section of the shaft, whereas the opposing end of the shaft is referred to as the drive-side section of the shaft.

In order to measure the applied pressure between the mating strips, a strain gauge beam may be installed across the two shafts. The strain gauge beam may be installed either at the outside section of the shafts or at the drive-side section of the shafts, or two strain gauges may be installed, one on each of the outside section and the drive-side section of the shafts. The advantage of installing strain gauge beams on both outside and drive-side sections of the shaft is that it can enable even finer process control.

The strain gauge beam(s) may also be used to make fine adjustments to the distance between the two shafts. This aspect of process control enables the apparatus to compensate for tool wear by finely adjusting the required distance between the mating strips to provide better quality assurance and reduce maintenance.

### Absorbent Material

The absorbent material 100 herein is preferably a flowable material, in the dry state, most preferably a particulate material. In particulate form the absorbent material may be particles, flakes, fibers, spheres, agglomerated particles and other forms known in the art.

The absorbent material 100 comprises superabsorbent polymer material, optionally combined with cellulosic material, including for example cellulose, comminuted wood pulp in the form of fibers. In some embodiments, the absorbent material 100 may comprise at least 60%, or at least 70% by weight of superabsorbent polymer material, and at the most 40% or at the most 30% of cellulosic material. In preferred embodiments, the absorbent layer comprises absorbent material 100 that consists substantially of absorbent polymer material, e.g. less than 5% by weight of the absorbent material of cellulosic material is present; and the absorbent layer/ absorbent structure, may be substantially or even completely free of cellulosic material.

In preferred embodiments herein, the absorbent material is a superabsorbent polymer material, herein referred to as SAP, and also known as particulate absorbent gelling material, AGM. The particulate SAP herein may have a high sorption capacity, e.g. having a CRC of for example at least 20 g/g, or at 30 g/g. Upper limits may for example be up to 150 g/g, or up to 100 g/g.

The particulate SAP may have a good permeability for liquid, for example, having a SFC value of at least 10 x 10⁻⁷ cm³ s/g; or preferably at least 30 x 10⁻⁷ cm³.s/g, or at least 50 x 10⁻⁷ cm³s/g 10 x 10⁻⁷ cm³s/g, or possibly permeability SFC value of at least 100 x10 ⁻⁷ cm³s/g, or at least a SFC of 120 x10⁻⁷ cm³sec/g. This SFC is a measure of permeability and an indication of porosity is provided by the saline flow conductivity of the gel bed as described in U.S. Patent No. 5,562,646, (Goldman et al.) issued Oct. 8, 1996 (whereby however a 0.9% NaCl solution is used instead of Jayco solution). Upper limits may for example be up to 350 or up to 250 (x 10⁻⁷ cm³.s/g).

In some embodiments herein the polymers of the SAP are internally cross-linked and/ or surface crosslinked polymers.

In some embodiments herein, the absorbent material 100 comprising or consisting of particles of polyacrylic acids/ polyacrylate polymers, for example having a neutralization degree of from 60% to 90%, or about 75%, having for example sodium counter ions, as known in the art; they may be surface crosslinked and/ or internally crosslinked polyacrylic acid/ polyacrylate polymers.

In some embodiments herein, the absorbent material 100 is in the form of particles with, a mass medium particle size up to 2 mm, or between 50 microns and 2 mm or to 1 mm, or preferably from 100 or 200 or 300 or 400 or 500µm, or to 1000 or to 800 or to 700 µm; as can for example be measured by the method set out in for example EP-A-0691133. In some embodiments of the invention, the material is in the form of particles of which at least 80% by weight are particles of a size between 50 µm and 1200 µm and having a mass median particle size between any of the range combinations above. The particles may be essentially spherical, or may have some other shape. Preferably the absorbent material 100 has a relatively narrow range of particle sizes, e.g. with the majority (e.g. at least 80% or preferably at least 90% or even at least 95% by weight) of particles having a particle size between 50µm and 1000µm, preferably between 100µm and 800µm, and more preferably between 200µm and 600µm.

The absorbent material 100 herein may advantageously comprise less than 15% by weight of water, or less than 10%, or less than 8% or less than 5%. The water-content can be determined by the Edana test, number ERT 430.1-99 (February 1999) which involves drying the particulate material 100 at 105°Celsius for 3 hours and determining the moisture content by the weight loss of the particulate material 100 after drying.

The particulate SAP herein may be particles of SAP that are surface coated or surface treated (this not including surface-crosslinking, which may be an additional surface-treatment); such coatings and surface treatment steps are well known in the art, and include surface treatment with one or more inorganic powders, including silicates, phosphates, and coatings of polymeric material, including elastomeric polymeric materials, or film-forming polymeric materials.

### Supporting sheet

The absorbent structure produced with the apparatus and method of the invention comprises a supporting sheet 16, to receive the absorbent material. This supporting sheet 16 may be any individual sheet or web sheet material, in particular paper, films, wovens or nonwovens, or laminate of any of these.

In some embodiments herein, the supporting sheet 16 is a nonwoven, e.g. a nonwoven web, such as a carded nonwoven, spunbond nonwoven or meltblown nonwoven, and including nonwoven laminates of any of these.

The fibers may be of natural or man-made origin and may be staple or continuous filaments or be formed in situ. Commercially available fibers have diameters ranging typically from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). The fibers may be bicomponent fibers, for example having a sheet-core arrangement, e.g. with different polymers forming the sheet and the core. Nonwoven fabrics can be formed by many processes such as meltblowing, spunbonding, solvent spinning, electrospinning, and carding.

The nonwoven herein may be made of hydrophilic fibers; "Hydrophilic" describes fibers or surfaces of fibers, which are wettable by aqueous fluids (e.g. aqueous body fluids) deposited on these fibers. Hydrophilicity and wettability are typically defined in terms of contact angle and the strike through time of the fluids, for example through a nonwoven fabric. This is discussed in detail in the American Chemical Society publication entitled "Contact angle, wettability and adhesion", edited by Robert F. Gould (Copyright 1964). A fiber or surface of a fiber is said to be wetted by a fluid (i.e. hydrophilic) when either the contact angle between the fluid and the fiber, or its surface, is less than 90°, or when the fluid tends to spread spontaneously across the surface of the fiber, both conditions are normally co-existing. Conversely, a fiber or surface of the fiber is considered to be hydrophobic if the contact angle is greater than 90° and the fluid does not spread spontaneously across the surface of the fiber.

The supporting sheet 16 herein may be air-permeable. Films useful herein may therefore comprise micro pores. Nonwovens herein may for example be air permeable. The supporting sheet 16 may have for example an air-permeability of from 40 or from 50, to 300 or to 200 m³/ (m²x min), as determined by EDANA method 140-1-99 (125 Pa, 38.3 cm²). The supporting sheet 16 may alternatively have a lower air-permeability, e.g. being non-air-permeable, to for example be better detained on a moving surface comprising vacuum.

In preferred executions, the supporting sheet 16 is a nonwoven laminate material, a nonwoven laminate web, for example of the SMS or SMMS type.

The basis weight of nonwoven fabrics is usually expressed in grams per square meter (gsm)., The supporting sheet 16 may have a basis weight that is less than 60 gsm, or for example than 50 gsm, for example from 5 gsm to 40 gsm, or to 30 gsm.

The supporting sheet 16 may have a transverse-extensibility or a longitudinal-extensibility, for example of more the 20 %, or for example more than 100 %, but for example not more than 200 %.

In one of the embodiment herein, the supporting sheet 16 has a transverse dimension that is more than the transverse dimension of the part of the receptacle (33), e.g. at least 10%, or for example at 20% or at least 30%, and for example up to about 120%.

### Adhesive application units and method steps.

The supporting sheet 16 is preferably treated with an adhesive prior to, at, and/or after transfer to the moving endless surface. Thus, the apparatusshown in Figure 2 comprises adhesive application unit(s) 136, 48, 146, 158. The method herein also comprises such an adhesive application step.

This adhesive may be applied uniformly and/or continuously, to aid absorbent material 100 immobilization and then it may help to adhere the supporting sheet 16 to a further material that may overlay the absorbent layer, as described below. Alternatively, it may be applied in a pattern. It may be applied by spraying, or for example by selectively slot-coating; the apparatus may thus comprise a slot-coater, with a pattern.

The adhesive may be applied on those portions of the supporting sheet 16 that are to receive to receive the absorbent material; then, it helps to immobilize the absorbent material 100 thereon (e.g. to ensure the absorbent material 100 will stay substantially as applied, with the channels, preferably not only during manufacturing, but also during storage and in use (at least during part of the use). Or, alternatively, only on those portions of the supporting sheet 16 that are to be on the mating strips 31; then it may help to adhere the supporting sheet 16 to a further material that may overlay the absorbent layer, as described below. It may be applied as substantially longitudinal stripes, for example.

In some embodiments, the apparatus may comprise a unit to apply an adhesive to the supporting sheet 16 in a pattern, for example the pattern of the mating strips 31, and optionally of the rods 36, if present.

Any suitable adhesive can be used for this, for example so-called hotmelt adhesives used, for example, sprayable hot melt adhesives, such as H.B. Fuller Co. (St. Paul, MN) Product No. HL-1620-B, can be used.

Alternatively, or in addition, it may be beneficial to apply a further immobilization adhesive to the absorbent structure produced by the apparatus or method herein, e.g. to ensure the absorbent material 100 will stay substantially as applied, with the channels, preferably not only during manufacturing, but also during storage and in use (at least during part of the use). This immobilization adhesive may then for example be applied onto the absorbent layer just after application of the absorbent material 100 onto the supporting sheet 16.

The apparatus herein may optionally have a further adhesive application unit 50, e.g. downstream from the absorbent material deposition point. The method may have a corresponding method step

This adhesive may be applied uniformly and/or homogeneously. This may be a thermoplastic adhesive material.

In accordance with certain embodiments, the thermoplastic adhesive material may comprise, in its entirety, a single thermoplastic polymer or a blend of thermoplastic polymers, having a softening point, as determined by the ASTM Method D-36-95 "Ring and Ball", in the range between 50 °C and 300 °C, or alternatively the thermoplastic adhesive material may be a hot melt adhesive comprising at least one thermoplastic polymer in combination with other thermoplastic diluents such as tackifying resins, plasticizers and additives such as antioxidants. In certain embodiments, the thermoplastic polymer has typically a molecular weight (Mw) of more than 10,000 and a glass transition temperature (Tg) usually below room temperature or -6 °C < Tg < 16°C. In certain embodiments, typical concentrations of the polymer in a hot melt are in the range of about 20 to about 40% by weight. In certain embodiments, thermoplastic polymers may be water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof. Other suitable thermoplastic polymers that may be employed are metallocene polyolefins, which are ethylene polymers prepared using single-site or metallocene catalysts. Therein, at least one comonomer can be polymerized with ethylene to make a copolymer, terpolymer or higher order polymer. Also applicable are amorphous polyolefins or amorphous polyalphaolefins (APAO) which are homopolymers, copolymers or terpolymers of C2 to C8 alpha olefins. In exemplary embodiments, the tackifying resin has typically a Mw below 5,000 and a Tg usually above room temperature, typical concentrations of the resin in a hot melt are in the range of about 30 to about 60%, and the plasticizer has a low Mw of typically less than 1,000 and a Tg below room temperature, with a typical concentration of about 0 to about 15%. In certain embodiments, the thermoplastic adhesive material is present in the form of fibers. In some embodiments, the fibers will have an average thickness of about 1 to about 50 micrometers or about 1 to about 35 micrometers and an average length of about 5 mm to about 50 mm or about 5mm to about 30 mm.

### Test Method

According to the apparatus and method of the present invention, an adhesive bond is formed between first and second supporting sheets in at least a part of the channel area. The bond strength of the adhesively connected regions may be measured by various test methods. A preferred test method is the peel strength test method as described in ASTM D-903-98. In case the standard peel rate of 152 mm (6 inches)/minute results in the tearing of the fragile supporting sheet, then this rate may need to be reduced. Peel strength is expressed in kilograms per millimeter of sample width (pounds per inch). 10 mm/minute is preferred.

One suitable power-driven machine for use in the test is a vertical single column Zwick Tensile Tester Test machine with 50 N force cell with force accuracy of 0.3 % and distance accuracy of 0.15 %. The four faces of the two jaws should be padded with a thin strip of rubber to prevent slippage as provided by the supplier of the equipment (jaws: Vulkollan glatt 30x60 mm, material number 314366, provider: Zwick GmbH & Co, D-89079 Ulm).

## Claims

1. An apparatus for making an absorbent structure for an absorbent article, comprising a first and second supporting sheet (16, 16') and therebetween an absorbent layer, the absorbent layer comprising an absorbent material (100), the apparatus comprising:
a) transfer means for transferring the first and second supporting sheets (16, 16') to first and second moving endless surfaces;
b) a feeder (156) for feeding the absorbent material (100) onto at least the first supporting sheet (16) at a depositing point on the first moving endless surface (30), the absorbent material (100) forming absorbent regions upon the first supporting sheet (16), and one or more channels between the absorbent regions, the channels being free of absorbent material (100);
c) an adhesive applicator (148) for applying adhesive to at least one of the first and second supporting sheets (16, 16'), at least within a region of the channels;
**characterized in that** the first moving endless surface (30) comprises one or more longitudinally extending first mating strips (31), and the second moving endless surface comprises corresponding longitudinally extending second mating strips, the first and second mating strips acting upon each other by applying pressure to the first and second supporting sheets (16, 16') at least within a part of an area of the channels, so as to adhere together the first and second supporting sheets (16, 16').

2. An apparatus according to claim 1 wherein the first mating strips (31) and the second mating strips are made from an elastic material, preferably silicone.

3. An apparatus according to either of claims 1 or 2, wherein the first moving endless surface (30), comprises an outer shell that comprises one or more air permeable or partially air permeable receptacle(s) (33) for receiving the first supporting sheet (16) thereon, and whereby the outer shell is connected to one or more vacuum systems for facilitating retention of the first supporting sheet (16) and/or the absorbent material (100) thereon.

4. An apparatus according to any of the preceding claims, whereby the receptacle (33) further comprise a multitude of longitudinally extending rods (36), spaced apart from one another in a transverse direction, each rod having a maximum width dimension of at least 0.3 mm and less than 2.5 mm, the rods (36) each having an average height dimension of at least 1 mm.

5. An apparatus according to any of the preceding claims, whereby the feeder (156) comprises a reservoir (25) formed by a multitude of cavities (22) and/or grooves.

6. An apparatus according to claim 5 whereby the cavities (22) and/or grooves that are directly adjacent a raised strip (21), have a volume that is more than a volume of one or more, or all of neighboring cavities (22) or grooves that are not directly adjacent the raised strip (21).

7. An apparatus according to any of the preceding claims, wherein the feeder (156) is a particulate superabsorbent polymer material feeder.

8. An apparatus according to any of the previous claims, comprising a second adhesive applicator (158) downstream from the depositing point.

9. A method for making an absorbent structure comprising a first and second supporting sheet (16, 16') and thereon an absorbent layer of absorbent material (100), the method comprising the steps of:
a) transferring first and second supporting sheets (16, 16') to first and second moving endless surfaces;
b) feeding the absorbent material (100) onto at least the first supporting sheet (16) at a depositing point on the first moving endless surface (30), the absorbent material (100) forming absorbent regions and one or more channels between the absorbent regions, the channels being free of absorbent material (100);
c) applying adhesive to at least one of the first and second supporting sheets (16, 16'), at least within a region of the channels;
**characterized in that** the first moving endless surface (30) comprises one or more longitudinally extending first mating strips (31), and the second moving endless surface (40) comprises corresponding longitudinally extending second mating strips, and wherein the method further comprises the step of applying pressure through the first and second mating strips to the first and second supporting sheets (16, 16') at least within a part of an area of the channels, so as to adhere together the first and second supporting sheets (16, 16').

10. A method according to claim 9, whereby the absorbent material (100) is a particulate superabsorbent polymer material.

11. A method according to either of claims 9 or 10, comprising the step of providing a first adhesive application unit (148), and applying an adhesive to the supporting sheet (16), prior to deposition of the absorbent material (100) thereon and/or comprising the step of providing a second adhesive application unit (158), and applying an adhesive to the absorbent structure prior to removing it from the first moving endless surface (30), or immediately subsequent thereto.

## Patentansprüche

1. Vorrichtung zur Herstellung einer Absorptionsstruktur für einen Absorptionsartikel, umfassend eine erste und eine zweite Stützschicht (16, 16') und dazwischen eine Absorptionsschicht, wobei die Absorptionsschicht ein Absorptionsstoff (100) umfasst, wobei die Vorrichtung Folgendes umfasst:
a) Übertragungsmittel zum Übertragen der ersten und der zweiten Stützschicht (16, 16') auf eine erste und eine zweite, sich bewegende endlose Oberfläche;
b) eine Zufuhrvorrichtung (156) zum Zuführen des Absorptionsstoffs (100) auf wenigstens die erste Stützschicht (16) an einer Ablagerungsstelle auf der ersten, sich bewegenden endlosen Oberfläche (30), wobei der Absorptionsstoff (100) Absorptionsbereiche auf der ersten Stützschicht (16) und einen oder mehrere Kanäle zwischen den Absorptionsbereichen ausbildet, wobei die Kanäle frei von Absorptionsstoff (100) sind;
c) einen Klebstoffapplikator (148) zum Auftragen von Klebstoff auf mindestens eine der ersten und der zweiten Stützschicht (16, 16') wenigstens innerhalb eines Bereichs der Kanäle;
**dadurch gekennzeichnet, dass** die erste, sich bewegende endlose Oberfläche (30) ein oder mehrere sich in Längsrichtung erstreckende, erste, zusammenpassende Streifen (31) umfasst, und die zweite, sich bewegende endlose Oberfläche entsprechende, sich in Längsrichtung erstreckende, zweite, zusammenpassende Streifen umfasst, wobei die ersten und zweiten zusammenpassenden Streifen aufeinander einwirken, indem Druck auf die erste und die zweite Stützschicht (16, 16') wenigstens innerhalb eines Teils eines Bereichs der Kanäle ausgeübt wird, um die erste und die zweite Stützschicht (16, 16') zusammenzukleben.

2. Vorrichtung nach Anspruch 1, wobei die ersten zusammenpassenden Streifen (31) und die zweiten zusammenpassenden Streifen aus einem elastischen Material, vorzugsweise Silikon, hergestellt sind.

3. Vorrichtung nach einem der Ansprüche 1 oder 2, wobei die erste sich bewegende endlose Oberfläche (30) eine äußere Schale umfasst, die einen oder mehrere luftdurchlässige oder teilweise luftdurchlässige Behälter (33) zum Aufnehmen der ersten Stützschicht (16) darauf umfassen, und wobei die äußere Schale mit einem oder mehreren Vakuumsystemen verbunden ist, um die Retention der ersten Stützschicht (16) und/oder des Absorptionsstoffs (100) darauf zu erleichtern.

4. Vorrichtung nach einem der vorstehenden Ansprüche, wobei der Behälter (33) ferner eine Mehrzahl von sich in Längsrichtung erstreckenden Stangen (36) umfasst, die in einer Querrichtung voneinander beabstandet sind, wobei jede Stange eine maximale Breitenabmessung von mindestens 0,3 mm und weniger als 2,5 mm aufweist, wobei die Stangen (36) jeweils eine durchschnittliche Höhenabmessung von mindestens 1 mm aufweisen.

5. Vorrichtung nach einem der vorstehenden Ansprüche, wobei die Zufuhrvorrichtung (156) ein Reservoir (25) umfasst, das durch eine Mehrzahl von Hohlräumen (22) und/oder Rillen gebildet ist.

6. Vorrichtung nach Anspruch 5, wobei die Hohlräume (22) und/oder Rillen, die direkt an einen erhöhten Streifen (21) angrenzen, ein Volumen aufweisen, das größer ist als ein Volumen eines oder mehrerer oder aller benachbarter Hohlräume (22) oder Rillen, die nicht direkt an den erhöhten Streifen (21) angrenzen.

7. Vorrichtung nach einem der vorstehenden Ansprüche, wobei die Zufuhrvorrichtung (156) eine Zufuhrvorrichtung für teilchenförmiges Superabsorber-Polymermaterial ist.

8. Vorrichtung nach einem der vorstehenden Ansprüche, umfassend einen zweiten Klebstoffapplikator (158), welcher der Anlagerungsstelle nachgeschaltet ist.

9. Verfahren zur Herstellung einer Absorptionsstruktur, umfassend eine erste und eine zweite Stützschicht (16, 16') und darauf eine Absorptionsschicht aus Absorptionsstoff (100), wobei das Verfahren die folgenden Schritte umfasst:
a) Übertragen der ersten und der zweiten Stützschicht (16, 16') auf eine erste und eine zweite, sich bewegende endlose Oberfläche;
b) Zuführen des Absorptionsstoffs (100) auf mindestens die erste Stützschicht (16) an einer Anlagerungsstelle auf der ersten, sich bewegenden endlosen Oberfläche (30), wobei der Absorptionsstoff (100) Absorptionsbereiche und einen oder mehrere Kanäle zwischen den Absorptionsbereichen ausbildet, wobei die Kanäle frei von Absorptionsstoff (100) sind;
c) Auftragen von Klebstoff auf mindestens eine der ersten und der zweiten Stützschicht (16, 16') wenigstens innerhalb eines Bereichs der Kanäle;
**dadurch gekennzeichnet, dass** die erste, sich bewegende endlose Oberfläche (30) ein oder mehrere sich in Längsrichtung erstreckende, erste, zusammenpassende Streifen (31) umfasst, und die zweite, sich bewegende endlose Oberfläche (40) entsprechende, sich in Längsrichtung erstreckende, zweite, zusammenpassende Streifen umfasst, und wobei das Verfahren ferner den Schritt des Ausübens von Druck durch die ersten und zweiten zusammenpassenden Streifen auf die erste und die zweite Stützschicht (16, 16') wenigstens innerhalb eines Teils eines Bereichs des Kanals umfasst, um die erste und die zweite Stützschicht (16, 16') zusammenzukleben.

10. Verfahren nach Anspruch 9, wobei der Absorptionsstoff (100) ein teilchenförmiges Superabsorber-Polymermaterial ist.

11. Verfahren nach einem der Ansprüche 9 oder 10, umfassend den Schritt des Bereitstellens einer ersten Klebstoffauftragseinheit (148) und des Auftragens eines Klebstoffs auf die Stützschicht (16) vor der Anlagerung des Absorptionsstoffs (100) darauf und/oder umfassend den Schritt des Bereitstellens einer zweiten Klebstoffauftragseinheit (158) und des Auftragens eines Klebstoffs auf die Absorptionsstruktur vor deren Entfernung von der ersten sich bewegenden endlosen Oberfläche (30) oder unmittelbar danach.

## Revendications

1. Appareil de fabrication d'une structure absorbante pour un article absorbant, comprenant des première et deuxième feuilles de soutien (16, 16') et, entre elles, une couche absorbante, la couche absorbante comprenant un matériau absorbant (100), l'appareil comprenant :
a) un moyen de transfert pour transférer les première et deuxième feuilles de soutien (16, 16') vers des première et deuxième surfaces en mouvement sans fin ;
b) un dispositif d'alimentation (156) pour alimenter le matériau absorbant (100) sur au moins la première feuille de soutien (16) au niveau d'un point de dépôt sur la première surface en mouvement sans fin (30), le matériau absorbant (100) formant des régions absorbantes sur la première feuille de soutien (16), et un ou plusieurs canaux entre les régions absorbantes, les canaux étant exempts de matériau absorbant (100) ;
c) un applicateur d'adhésif (148) pour appliquer un adhésif sur au moins une des première et deuxième feuilles de soutien (16, 16'), au moins au sein d'une région des canaux ;
**caractérisé en ce que** la première surface en mouvement sans fin (30) comprend une ou plusieurs premières bandes d'accouplement s'étendant longitudinalement (31), et la deuxième surface en mouvement sans fin comprend des deuxièmes bandes d'accouplement s'étendant longitudinalement correspondantes, les premières et deuxièmes bandes d'accouplement agissant l'une sur l'autre en appliquant une pression aux première et deuxième feuilles de soutien (16, 16') au moins au sein d'une partie d'une zone des canaux, de façon à faire adhérer l'une à l'autre les première et deuxième feuilles de soutien (16, 16').

2. Appareil selon la revendication 1, dans lequel les premières bandes d'accouplement (31) et les deuxièmes bandes d'accouplement sont fabriquées à partir d'un matériau élastique, de préférence de la silicone.

3. Appareil selon la revendication 1 ou la revendication 2, dans lequel la première surface en mouvement sans fin (30) comprend une enveloppe externe qui comprend un ou plusieurs réceptacle(s) (33) perméable(s) à l'air ou partiellement perméable(s) à l'air pour recevoir la première feuille de soutien (16) sur ceux-ci, et selon lequel l'enveloppe externe est reliée à un ou plusieurs systèmes de vide pour faciliter la rétention de la première feuille de soutien (16) et/ou du matériau absorbant (100) sur ceux-ci.

4. Appareil selon l'une quelconque des revendications précédentes, selon lequel le réceptacle (33) comprend en outre une multitude de tiges s'étendant longitudinalement (36), espacées les unes des autres dans une direction transversale, chaque tige possédant une dimension en largeur maximale d'au moins 0,3 mm et inférieure à 2,5 mm, les tiges (36) ayant chacune une dimension en hauteur moyenne d'au moins 1 mm.

5. Appareil selon l'une quelconque des revendications précédentes, selon lequel le dispositif d'alimentation (156) comprend un réservoir (25) formé par une multitude de cavités (22) et/ou de rainures.

6. Appareil selon la revendication 5, selon lequel les cavités (22) et/ou rainures qui sont directement adjacentes à une bande relevée (21), ont un volume qui est supérieur à un volume d'une ou plusieurs, ou de la totalité des cavités (22) ou rainures voisines qui ne sont pas directement adjacentes à la bande relevée (21).

7. Appareil selon l'une quelconque des revendications précédentes, dans lequel le dispositif d'alimentation (156) est un dispositif d'alimentation de matériau polymère superabsorbant particulaire.

8. Appareil selon l'une quelconque des revendications précédentes, comprenant un deuxième applicateur d'adhésif (158) en aval du point de dépôt.

9. Procédé de fabrication d'une structure absorbante comprenant des première et deuxième feuilles de soutien (16, 16') et sur celle-ci une couche absorbante de matériau absorbant (100), le procédé comprenant les étapes consistant à :
a) transférer des première et deuxième feuilles de soutien (16, 16') vers des première et deuxième surfaces en mouvement sans fin ;
b) alimenter le matériau absorbant (100) sur au moins la première feuille de soutien (16) au niveau d'un point de dépôt sur la première surface en mouvement sans fin (30), le matériau absorbant (100) formant des régions absorbantes et un ou plusieurs canaux entre les régions absorbantes, les canaux étant essentiellement exempts de matériau absorbant (100) ;
c) appliquer un adhésif sur au moins une des première et deuxième feuilles de soutien (16, 16'), au moins au sein d'une région des canaux ;
**caractérisé en ce que** la première surface en mouvement sans fin (30) comprend une ou plusieurs premières bandes d'accouplement s'étendant longitudinalement (31), et la deuxième surface en mouvement sans fin (40) comprend des deuxièmes bandes d'accouplement s'étendant longitudinalement correspondantes, et où le procédé comprend en outre l'étape d'application de pression à travers les première et deuxième bandes d'accouplement sur les première et deuxième feuilles de soutien (16, 16') au moins au sein d'une partie d'une zone des canaux, de façon à faire adhérer l'une à l'autre les première et deuxième feuilles de soutien (16, 16').

10. Procédé selon la revendication 9, selon lequel le matériau absorbant (100) est un matériau polymère superabsorbant particulaire.

11. Procédé selon la revendication 9 ou la revendication 10, comprenant l'étape de fourniture d'une première unité d'application d'adhésif (148), et d'application d'un adhésif sur la feuille de soutien (16), avant le dépôt du matériau absorbant (100) sur celle-ci et/ou comprenant l'étape de fourniture d'une deuxième unité d'application d'adhésif (158), et d'application d'un adhésif sur la structure absorbante avant son retrait de la première surface en mouvement sans fin (30), ou immédiatement à la suite de celui-ci.
